(19) Europäisches Patentamt
European Patent Office
Office européen des brevets

(11) EP 0 815 847 B1

(12) # FASCICULE DE BREVET EUROPEEN

(45) Date de publication et mention de la délivrance du brevet: **14.04.1999 Bulletin 1999/15**

(51) Int. Cl.$^6$: **A61K 7/48**, A61K 7/16

(21) Numéro de dépôt: **97401257.7**

(22) Date de dépôt: **04.06.1997**

(54) **Composition cosmétique et/ou dermatologique contenant au moins un précurseur d'actif et un poly(acide 2-acrylamido 2-méthylpropane sulfonique) réticulé**

Kosmetische und/oder dermatologische Zusammensetzung, die mindestens einen Wirkstoffvorläufer und mindestens eine vernetzte Poly(2-acrylamido-2-methylpropansulfonsäure) enthält

Cosmetic and/or dermatological composition containing at least an active agent precursor and a crosslinked polymer of 2-acrylamide-2-methylpropane sulfonic acid

(84) Etats contractants désignés:
**DE ES FR GB IT**

(30) Priorité: **28.06.1996 FR 9608110**

(43) Date de publication de la demande:
**07.01.1998 Bulletin 1998/02**

(73) Titulaire: **L'OREAL**
**75008 Paris (FR)**

(72) Inventeurs:
- **Sebillotte-Arnaud, Laurence**
  **94240 L'Hay Les Roses (FR)**
- **Lorant, Raluca**
  **94320 Thiais (FR)**

(74) Mandataire: **Miszputen, Laurent**
**L'Oreal,**
**DPI,**
**6 rue Sincholle**
**92585 Clichy Cédex (FR)**

(56) Documents cités:
**EP-A- 0 487 404**
**EP-A- 0 669 126**
**US-A- 5 114 706**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 815 847 B1

Printed by Xerox (UK) Business Services
2.16.7/3.6

## Description

L'invention se rapporte à une composition cosmétique et/ou dermatologique contenant au moins un précurseur d'actif apte à libérer un actif par réaction enzymatique au contact du *Stratum Corneum* et au moins un polymère poly(acide 2-acrylamido 2-méthylpropane sulfonique) réticulé et neutralisé à au moins 90% ainsi qu'à ses utilisations.

Depuis un certain nombre d'années, on cherche, de plus en plus, à introduire dans des compositions cosmétiques et/ou dermatologiques des vitamines comme les vitamines A, B, C, D, E, F ainsi que d'autres actifs en vue d'apporter des traitements spécifiques contre notamment les surcharges pondérales, le vieillissement de la peau, son dessèchement, sa pigmentation, l'acné et certaines maladies de peau (psoriasis), ou encore pour notamment favoriser la cicatrisation et/ou la restructuration de la peau.

En particulier, l'application d'acide ascorbique ou vitamine C sur la peau, en quantité suffisante, permet de stimuler la croissance du tissu conjonctif et notamment celle du collagène. L'acide ascorbique permet également de renforcer les défenses du tissu cutané contre les agressions extérieures telles que les rayonnements ultraviolets ou la pollution, contre les agressions des médicaments, de l'alcool, ou du tabac.

Par ailleurs, les tocophérols tels que la vitamine E sont connus pour posséder, à la fois, des propriétés antioxydantes vis-à-vis des phospholipides de la membrane cellulaire, et des propriétés anti-radicalaires (ARL) ( voir "Radicaux libres et Vitamine E" de J.B. CHAZAN et M.SZULC -Cah. Nutr. Diet. 1987 6 XXII - 1 - pages 66-76).

En outre, la vitamine A ou rétinol, ainsi que les hydroxyacides sont connues pour lutter contre le vieillissement. De plus la vitamine A est connue pour assurer la cicatrisation de la peau.

Malheureusement, la plupart de ces actifs (vitamines, antioxydants, hydroxyacides etc.) est instable en solution, sensible à des facteurs extérieurs rendant ces solutions inopérantes et allant à l'encontre de l'efficacité recherchée.

En particulier, l'article "Stability of ascorbic acid" de BR. HAJRATWALA paru dans "Sciences Pharmaceutiques Revue" pages 281-286, enseigne que l'acide ascorbique possède des propriétés d'instabilité en milieu aqueux, en milieu aérobie et anaérobie, avec une instabilité plus prononcée en milieu aérobie. Il y est illustré notamment le comportement de l'acide ascorbique face à des variations du pH de la solution le contenant, des variations de lumière, de température, face à des composés tels que des tensio-actifs, des solvants, des catalyseurs notamment métalliques.

Aussi, différents moyens ont été envisagés pour stabiliser l'acide ascorbique.

Parmi ces moyens, les brevets japonais JP 89/115 558 et JP 83/129 892 enseignent le blocage du site réactif de l'acide ascorbique, à savoir le site hydroxyle, par estérification et/ou éthérification avec notamment des dérivés phosphatés, sulfatés, alkylés et l'emploi de ces dérivés dans des compositions cosmétiques pour jouer le rôle de la vitamine C. Malheureusement, ces derniers sont beaucoup moins efficaces que la vitamine C libre (c'est-à-dire sans groupements additionnels).

A cet effet, on a envisagé l'emploi d'un précurseur de la vitamine C. Ainsi, le brevet européen EP 487 404 divulgue l'utilisation d'un dérivé glucosylé, dans des compositions dermatologiques, apte à libérer de l'acide ascorbique lorsque ces dernières sont mises au contact de la peau.

Le document EP-A-669 126 décrit une composition contenant un hydratant et un composé phosphaté apte à libérer sur la peau un actif différent de l'hydratant.

Par ailleurs, on a envisagé l'estérification d'un dérivé d'acide ascorbique et d'un dérivé de tocophérol avec l'acide phosphorique (voir le document "Bioconversion of a vitamin to vitamins C and E in skin" de KAKUJI TOJO et AE-RIC. LEE publié dans J. Cosmet. Chem., 38, pages 333 - 339) et son utilisation dans une composition.

Mais ce di-ester vis-à-vis de l'acide ascorbique présente une efficacité plus faible que celle de l'acide ascorbique libre et, vis-à-vis de la vitamine E, une activité anti-oxydante moins bonne que celle de la vitamine E libre.

Ce même problème se rencontre aussi pour tout type d'actif.

Par conséquent, il subsiste le besoin d'une composition cosmétique et/ou dermatologique stable qui permet la libération, avec un bon rendement, à partir d'un précurseur, d'une quantité importante d'actif par hydrolyse enzymatique, au contact du *Stratum corneum*.

D'autre part, les compositions cosmétiques ou dermatologiques présentent en général une viscosité élevée et sont pour la plupart formulées sous une forme liquide épaisse telle qu'un lait, une crème, un gel ou une pâte. Ce type de présentation est très appréciée par le consommateur ; il correspond le plus souvent à une préoccupation pratique pour le formulateur : faciliter la prise du produit hors de son conditionnement sans perte significative, limiter la diffusion du produit à la zone locale de traitement et pouvoir l'utiliser dans des quantités suffisantes pour obtenir l'effet cosmétique ou dermatologique recherché.

Cet objectif est important pour les formulations telles que celles des produits pour le soin, l'hygiène ou le maquillage qui doivent bien s'étaler de façon homogène sur la surface locale à traiter.

Pour satisfaire à ces conditions, on augmente la viscosité des compositions par l'ajout de polymères épaississants et/ou gélifiants.

Les précurseurs d'actifs tels que décrits précédemment peuvent difficilement être incorporés dans des formulations cosmétiques ou dermatologiques en présence des polymères épaississants et/ou gélifiants couramment utilisés. Ces polymères au contact desdits précurseurs d'actif perdent en général leur pouvoir épaississant et/ou gélifiant et ne permettent pas d'obtenir des formulations de viscosité élevée et stable. D'autre part, l'augmentation de la concentration en polymère épaississant, en vue de stabiliser la formulation à base de précurseurs d'actif, conduit le plus souvent à des effets indésirables sur le plan cosmétique tels qu'un effet collant ou un aspect peu esthétique.

On connaît dans l'état de la technique des homopolymères poly(acide 2-acrylamido 2-méthylpropane sulfonique) comme les produits commerciaux COSMEDIA HSP1160 et RHEOTIK 8011 de la société HENKEL. Ils sont utilisés comme agents épaississants et/ou gélifiants dans de nombreuses formulations cosmétiques. Ces polymères ne permettent pas d'obtenir, en présence de précurseurs d'actif, des compositions stables et homogènes pouvant atteindre des viscosités élevées dans une large gamme de pH. Ils conduisent le plus souvent à des gels fluides, hétérogènes, filants et collants.

La demanderesse a découvert de manière surprenante une nouvelle famille de polymères épaississants et/ou gélifiants permettant d'obtenir des formulations cosmétiques et dermatologiques épaissies stables, à base de précurseurs d'actifs, permettant de traiter les matières kératiniques de façon efficace tout en assurant également un bon rendement de l'hydrolyse enzymatique du précurseur de l'actif.

D'autre part, ces polymères particuliers permettent de réaliser des produits cosmétiques et/ou dermatologiques homogènes pouvant atteindre des viscosités élevées et stables dans le temps à température ambiante ou à des températures plus élevées.

Enfin, ils permettent de réaliser des gels transparents, non-coulants, non-filants, doux et glissants à l'application.

Ainsi, l'invention a pour objet une à une composition cosmétique et/ou dermatologique contenant dans un milieu cosmétiquement acceptable au moins un précurseur d'actif apte à libérer un actif par réaction enzymatique au contact du *Stratum Corneum* et au moins un poly(acide 2-acrylamido 2-méthylpropane sulfonique) réticulé et neutralisé à au moins 90%.

La composition de l'invention a une certaine consistance et/ou tenue ; elle n'est pas filante, c'est-à-dire qu'elle ne forme pas de fil lorsqu'on la prend au doigt. Elle se présente plus spécialement sous forme d'un gel.

Les polymères poly(acide 2-acrylamido 2-méthylpropane sulfonique) réticulés et pratiquement ou totalement neutralisés, conformes à l'invention, sont hydrosolubles ou gonflables dans l'eau. Ils sont en général caractérisés par le fait qu'ils comprennent, distribués de façon aléatoire :

a) de 90 à 99,9% en poids de motifs de formule générale (1) suivante :

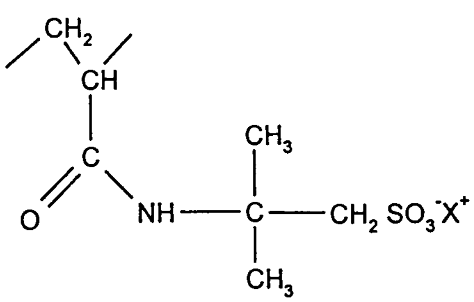

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

Les polymères selon l'invention plus particulièrement préférés comprennent de 98 à 99,5 % en poids de motifs de formule (1) et de 0,2 à 2 % en poids de motifs réticulants.

De façon préférentielle, les polymères de l'invention comportent un nombre de motifs de formule (1) dans une quantité suffisamment élevée pour obtenir un volume hydrodynamique du polymère en solution d'eau ayant un rayon allant de 10 à 500 nm, de distribution homogène et unimodale.

Les polymères selon l'invention plus particulièrement préférés comprennent de 98 à 99,5 % en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons

($H^+$).

Les monomères de réticulation ayant au moins deux doubles-liaisons oléfiniques sont choisis par exemple parmi le dipropylèneglycol-diallyléther, les polyglycol-diallyléthers, le triéhylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles-liaisons oléfiniques sont plus particulièrement choisis parmi ceux répondant à la formule générale (2) suivante :

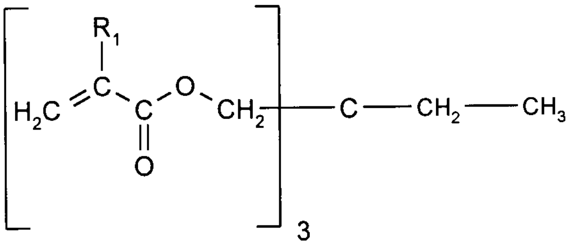

(2)

dans laquelle $R_1$ désigne un atome d'hydrogène ou un alkyle en $C_1$-$C_4$ et plus particulièrement l'hydrogène (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec :

M désignant la masse en grammes de la macromolécule non-dissoute ;

$N_A$ désignant le nombre d'Avogadro ;

$V_1$ désignant le volume spécifique du solvant ;

$V_2$ désignant le volume spécifique de la macromolécule ; d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution, on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al , Macromolecules, 1995, 28,4914-4919.

Les polymères particulièrement préférés sont ceux présentant une viscosité mesurée au viscosimètre BROOKFIELD dans une solution d'eau à 2% et à 25°C supérieure ou égale à 1000 mPa.s (1000 cPs) et plus préférentiellement allant de 5000 à 40000 mPa.s (5000 à 40000 cPs) et plus particulièrement de 6500 à 35000 mPa.s (6500 à 35000 cPs).

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol ;

(b) on neutralise la solution ou la dispersion de monomère AMPS obtenue en (a) par une ou plusieurs bases minérales ou organiques, de préférence l'ammoniaque $NH_3$ , dans une quantité permettant d'obtenir un taux de neutralisation des fonctions acides sulfoniques du polymère allant de 90 à 100% ;

(c) on ajoute à la solution ou dispersion obtenue en (b) le ou les monomères réticulants ;

(d) on effectue une polymérisation radicalaire classique en la présence d'amorceurs de radicaux libres à une température allant de 10 à 150°C ; le polymère précipitant dans la solution ou la dispersion à base de tertio-butanol.

Les poly(acide 2-acrylamido 2-méthylpropane sulfonique) réticulés, pratiquement ou totalement neutralisés sont présents dans les compositions cosmétiques ou dermatologiques de l'invention dans des concentrations allant préférentiellement de 0,01 à 20% en poids par rapport au poids total de la composition et plus préférentiellement de 0,1 à 10% en poids.

Selon l'invention, on peut choisir les précurseurs d'actif parmi les phosphates ; les sulfates ; les esters alkylés ou acylés d'actif ; les éthers acylés ou alkylés ; les amides d'actif et les dérivés d'ose d'actif. Ils peuvent être utilisés seuls ou en mélanges.

Les radicaux acylés et alkylés ont en particulier de 1 à 30 atomes de carbone. En particulier, les esters et les éthers alkylés ou acylés peuvent être issus de la réaction avec un acide minéral comme un sulfate ou un phosphate pour réagir avec une sulfatase ou phosphatase au contact de la peau, ou bien issus de la réaction avec un acide organique comme l'acide palmitique, acétique, propionique, nicotinique, 1,2,3 propane tricarboxylique, férulique pour réagir avec une estérase spécifique de la peau.

Selon l'invention, les précurseurs aptes à libérer un actif par hydrolyse enzymatique au contact de la peau, sont choisis de préférence parmi les précurseurs de vitamine tels que ceux de la vitamine A (rétinol), de la vitamine B, de la vitamine C (acide ascorbique), de la vitamine D, de la vitamine E (tocophérol), la vitamine F ; les précurseurs d'hydroxyacide tels que ceux de l'acide lactique, de l'acide glycolique ; les précurseurs de quercétine ; les précurseurs de nucléotide ; les hydroxyacétones phosphatées ; les précurseurs de glycérol ainsi que leurs mélanges.

A titre d'exemple pour les vitamines, on peut citer comme précurseurs les phosphates, les sulfates, un palmitate, un acétate, un nicotinate ou un propionate et les dérivés d'ose de vitamine A (rétinol), C (acide ascorbique) ou E (tocophérol).

Comme phosphate d'acide ascorbique, on peut utiliser l'ascorbyl phosphate d'un métal alcalin, alcalino terreux ou de transition comme le magnésium, le sodium, le potassium, le calcium, le zinc. Comme phosphate de rétinol, on peut utiliser le rétinyl phosphate d'un métal alcalin ou alcalino terreux comme le magnésium, le potassium.

Comme ester d'acide organique de la vitamine C, on peut utiliser un ester palmitique, acétique ou propionique greffé en position 2 ou 3 de la vitamine C. Comme ester de tocophérol, on peut utiliser les nicotinates ou acétates de tocophérol. Parmi les esters de rétinol, on peut utiliser un ester d'acide palmitique, propionique, acétique.

Parmi les dérivés d'ose de vitamine C utilisables dans l'invention, on peut citer à titre d'exemple la vitamine C glucosylé, mannosylé, fructosylé, N-acétylglucosaminé, le dérivé N-acétylmuramique de vitamine C, le dérivé fucosylé, galactosylé ou leurs mélanges.

Par ailleurs, à titre d'exemple pour l'acide lactique ($\alpha$-hydroxyacide), on peut citer le trilactate de glycérol, le lactate d'éthyle et les dérivés sulfatés de l'acide lactique.

Par ailleurs, à titre d'exemple pour le glycérol, on peut citer le trilactate de glycérol et les $\beta$-glycérophosphates pour libérer, au contact de la peau, le glycérol.

Toujours à titre d'exemple, on peut citer comme précurseurs de la quercétine, le glucosyle de quercétine et un ester de quercétine comme le férulate de quercétine pour libérer de façon synergique la quercétine.

Parmi les précurseurs de nucléotide, on peut citer l'adénosine phosphate, la guanosine phosphate, la cytosine phosphate, l'uridine phosphate, la thymidine phosphate, l'inosine phosphate, la xanthosine phosphate.

Les précurseurs d'actifs particulièrement préférés de l'invention sont les dérivés phosphatés de la vitamine C et plus préférentiellement les sels d'ascorbylphosphate de métal de transition comme le magnésium, le sodium, le potassium, le calcium, le zinc.

Le ou les précurseurs d'actif sont présents dans des concentrations allant de préférence de 0,01% à 10 % en poids, et plus préférentiellement de 0,01 % à 1 % en poids par rapport au poids total de la composition.

Les compositions de l'invention contiennent un milieu aqueux cosmétiquement acceptable, c'est-à-dire un milieu compatible avec toutes les matières kératiniques telles que la peau, les ongles, les muqueuses et les cheveux ou toute autre zone cutanée du corps.

Les compositions contiennent de préférence un milieu aqueux cosmétiquement et/ou dermatologiquement acceptable. Elles présentent un pH pouvant aller de préférence de 1 à 13 et plus préférentiellement de 2 à 12.

Le milieu cosmétiquement et/ou dermatologiquement acceptable des compositions selon l'invention est plus particulièrement constitué d'eau et éventuellement de solvants organiques cosmétiquement et/ou dermatologiquement acceptables (tolérance, toxicologie et toucher acceptables).

Les solvants organiques peuvent représenter de 5 % à 98 % du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des mono-alcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer des polyols tels que des dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Afin que les compositions cosmétiques ou dermatologiques de l'invention soient plus agréables à utiliser (plus douce à l'application, plus nourrissante, plus émolliente), il est possible d'ajouter une phase grasse dans le milieu de ces composition.

La phase grasse représente de préférence, de 0 % à 50 % du poids total de la composition.

Cette phase grasse peut comporter une ou plusieurs huiles choisies de préférence dans le groupe constitué par:

- les silicones volatiles ou non-volatiles, linéaires, ramifiées ou cycliques, organomodifiées ou non, hydrosolubles ou non hydrosolubles,
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées,
- les esters d'acides gras tels que l'huile de Purcellin.

Elle peut aussi comporter comme matière grasse un(e) ou plusieurs alcool gras, acides gras (acide stéarique) ou cires (paraffine, cires de polyéthylène, carnauba, cire d'abeilles).

De façon connue, toutes les compositions de l'invention peuvent contenir des adjuvants habituels dans les domaines cosmétique et dermatologique, d'autres gélifiants et/ou épaississants classiques aqueux ou lipophiles ; des actifs hydrophiles ou lipophiles ; des conservateurs ; des antioxydants ; des parfums ; des émulsionnants ; des agents hydratants ; des émollients ; des séquestrants ; des tensio-actifs ; des polymères ; des agents alcanisants ou acidifiants ; des charges ; des agents anti-radicaux libres ; des céramides ; des filtres solaires (notamment ultraviolets), des répulsifs pour insectes; des agents amincissants. ; des matières colorantes ; des bactéricides ; des antipelliculaires. Les quantités de ces différents adjuvants sont celles classiquement utilisées dans les domaines considérés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de solutions du type lotion ou sérum, sous forme de gels aqueux, sous forme d'émulsions obtenues par dispersion d'une phase grasse dans une phase aqueuse (H/E) ou inversement (E/H), de consistance liquide, semi-liquide ou solide telles que des laits, des crèmes plus ou moins onctueuses, des pâtes. Ces compositions sont préparées selon les méthodes usuelles.

Les compositions selon l'invention peuvent être utilisées comme produits rincés ou comme produits non-rincés capillaires notamment pour le lavage, le soin, le conditionnement, le maintien de la coiffure ou la mise en forme des fibres kératiniques telles que les cheveux.

Elles peuvent être des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux.

Les compositions de l'invention peuvent être également des shampooings, des compositions à rincer ou non, à appliquer avant ou après un shampooing, une coloration, une décoloration, une permanente ou un défrisage.

Les compositions de l'invention peuvent être également utilisées comme produit de soin et/ou l'hygiène tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions de l'invention peuvent être également utilisées comme produit antisolaire.

Les compositions peuvent également consister en des préparations solides constituant des savons ou des pains de nettoyage.

Les compositions de l'invention peuvent être également utilisées comme produit de soin bucco-dentaire tel que des pâtes dentifrices.

Les compositions peuvent être des produits pour le maquillage.

Un autre objet de l'invention est un procédé de traitement non-thérapeutique cosmétique de la peau, du cuir chevelu, des cheveux, des cils, des sourcils, des ongles ou des muqueuses notamment pour lutter contre les comédons chez les peaux grasses, pour favoriser la croissance des cheveux, pour prévenir le vieillissement de la peau, son dessèchement, sa pigmentation, pour favoriser la cicatrisation et/ou la restructuration de la peau, pour stimuler la croissance des tissus conjonctifs de l'épiderme. Il est caractérisé par le fait qu'on applique sur le support kératinique une composition telle que définie ci-dessus, selon la technique d'utilisation habituelle de cette composition. Par exemple : application de crèmes, de gels, de sérums, de lotions, de laits sur la peau, le cuir chevelu et/ou les muqueuses. Le type de traitement est fonction du ou des précurseurs d'actif présents dans la composition.

L'invention a encore pour objet une utilisation de la composition ci-dessus pour préparer une lotion, un sérum un lait, une pommade ou un onguent destiné à traiter thérapeutiquement les matières kératiniques telles que la peau, le cuir chevelu ou les muqueuses notamment pour lutter contre l'acné et les comédons chez les peaux grasses, contre la chute des cheveux, contre certaines maladies de la peau (psoriasis), contre le vieillissement de la peau, son dessèchement, sa pigmentation, pour favoriser la cicatrisation et/ou la restructuration de la peau, pour stimuler la croissance des tissus conjonctifs de l'épiderme.

Les exemples suivants illustrent l'invention sans présenter un caractère limitatif.

## EXEMPLE DE PREPARATION A

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant viscosité mesurée au viscosimètre BROOKFIELD, mobile 4, à une vitesse de rotation de 100 tours/minute dans une solution d'eau à 2 % et à 25 °C allant de 15000 mPa.s à 35000 mPa.s (15000 cPs à 35000 cPs). La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

**EXEMPLE DE PREPARATION B**

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minute dans une solution d'eau à 2 % et à 25°C de l'ordre de 7000 mPa.s (7000 cPs).

Le rayon hydrodynamique du polymère obtenu dans une solution aqueuse déterminé par diffusion de la lumière dynamique est de 160 nm.

**EXEMPLE 1 : Gel blanchissant**

| | |
|---|---|
| - Poly(acide 2-acrylamido 2-méthylpropane sulfonique) réticulé et neutralisé par de l'ammoniaque, préparé selon le procédé de l'exemple de préparation B de viscosité de l'ordre de 7000 mPa.s (7000 cPs) dans une solution d'eau à 2% et à 25°C | 1,5 % MA |
| - Ascorbylphosphate de magnésium | 0,3% |
| - Conservateur qs | |
| - Eau distillée qsp | 100 % |

On obtient un gel stable, épais, onctueux et homogène.

**EXEMPLE 2 : Gel fraîcheur pour peaux grasses**

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16000 mPa.s (16000 cPs) dans une solution d'eau à 2% et à 25°C | 2 g MA |
| - Ascorbyl phosphate de magnésium | 1 g |
| - Glycérine | 3 g |
| - Conservateur qs | |
| - Eau distillée qsp | 100 g |

On obtient un gel épais, parfaitement transparent, doux et frais sur la peau, non-coulant, non-filant à la prise.

**COMPARAISON AVEC UNE COMPOSITION DE L'ART ANTERIEUR**

En remplaçant le polymère Poly(acide 2-acrylamido 2-méthylpropane sulfonique) réticulé par un homopolymère d'acide 2-acrylamido 2-méthylpropane sulfonique non réticulé comme le produit commercial COSMEDIA HSP1160 vendu par HENKEL, on obtient un produit dont les propriétés cosmétiques sont rédhibitoires : aspect fluide, coulant, filant à la prise, poisseux au toucher.

**EXEMPLE 3 : Crème-gel de soin vitamine**

*Phase grasse*

| | |
|---|---|
| - Huile d'amandes douces | 4,0 g |
| - Cyclométhicone | 2,0 g |
| - Acétate de tocophérol | 1,0 g |

*Phase aqueuse*

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16000 mPa.s (16000 cPs) dans une solution d'eau à 2% et à 25°C | 1,5 g MA |
| - Ascorbyl phosphate de magnésium | 1,0 g |
| - Glycérine | 3,0 g |
| - Conservateur qs | |
| - Eau distillée qsp | 100 g |

On obtient une crème assez épaisse, non-coulante, lisse et brillante de pH 7.

**COMPARAISON AVEC UNE COMPOSITION DE L'ART ANTERIEUR**

En remplaçant le polymère Poly(acide 2-acrylamido 2-méthylpropane sulfonique) réticulé par un homopolymère d'acide 2-acrylamido 2-méthylpropane sulfonique non réticulé comme le produit commercial COSMEDIA HSP1160 vendu par HENKEL, on obtient une dispersion grossière, instable et d'aspect hétérogène. Quelle que soit la concentration en homopolymère, il n'a pas été possible de réaliser des produits épais et non-coulant.

**EXEMPLE 4 : Crème-lissante pour le visage**

*Phase grasse*

| | |
|---|---|
| - Huile de soja | 7,0 g |
| - Polyisobutène hydrogéné | 5,0 g |
| - Acétate de tocophérol | 0,5 g |

*Phase aqueuse*

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16000 mPa.s (16000 cPs) dans une solution d'eau à 2% et à 25°C | 1,5 g MA |
| - Acide glucopyranosyl L-ascorbyl | 1,0 g |
| - Glycérine | 3,0 g |
| - Conservateur qs | |
| - Eau distillée qsp | 100 g |

On obtient une crème blanche, lisse et brillante de pH 2,7.

**Revendications**

1. Composition cosmétique et/ou dermatologique, caractérisée par le fait qu'elle contient dans un milieu cosmétiquement acceptable au moins un précurseur d'actif apte à libérer un actif par réaction enzymatique au contact du *Stratum corneum* et au moins un poly(acide 2-acrylamido 2-méthylpropane sulfonique) réticulé et neutralisé à au moins 90%, comprenant, distribués de façon aléatoire :

   a) de 90 à 99,9% en poids de motifs de formule générale (1) suivante :

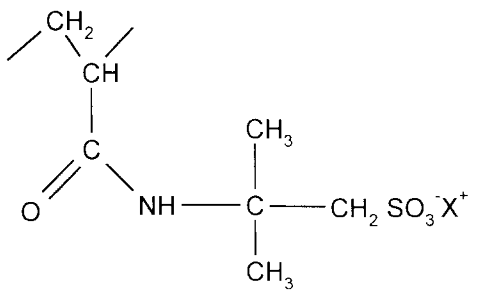

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

2. Composition selon la revendication 1, caractérisée par le fait que le poly(acide 2-acrylamido 2-méthylpropane sulfonique) réticulé comporte un nombre de motifs de formule (1) dans une quantité suffisamment élevée pour obtenir un volume hydrodynamique du polymère en solution d'eau ayant un rayon allant de 10 à 500 nm, de distribution homogène et unimodale.

3. Composition selon l'une quelconque des revendications 1 et 2, caractérisée par le fait que le poly(acide 2-acrylamido 2-méthylpropane sulfonique) réticulé comporte de 98 à 99,5% en poids de motifs de formule (1) et de 0,2 à 2% en poids de motifs réticulants.

4. Composition selon l'une quelconque des revendications 1 à 3, caractérisée par le fait que dans la formule (1) le cation $X^+$ est $NH_4^+$.

5. Composition selon l'une quelconque des revendications 1 à 4, caractérisée par le fait que les monomères réticulants répondent à la formule générale (2) suivante :

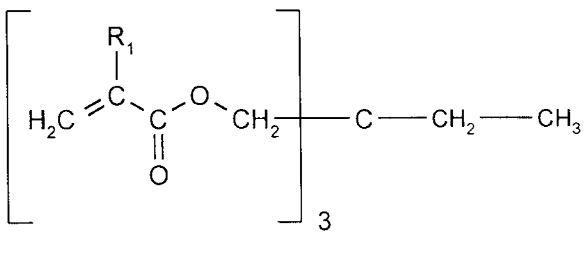

(2)

dans laquelle $R_1$ désigne un atome d'hydrogène ou un alkyle en $C_1$-$C_4$.

6. Composition selon l'une quelconque des revendications 1 à 5, caractérisée par le fait que le poly(acide 2-acrylamido 2-méthylpropane sulfonique) est réticulé par le triméthylol propane triacrylate.

7. Composition selon l'une quelconque des revendications 1 à 6, caractérisée par le fait que le poly(acide 2-acrylamido 2-méthylpropane sulfonique) réticulé présente une viscosité mesurée au viscosimètre BROOKFIELD, mobile 4, à une vitesse de rotation de 100 tours/minute, dans une solution d'eau à 2 % et à 25 °C supérieure ou égale à 1000 mPa.s (1000 cPs).

8. Composition selon l'une quelconque des revendications 1 à 7, caractérisée par le fait que le poly(acide 2-acrylamido 2-méthylpropane sulfonique) réticulé présente une viscosité mesurée au viscosimètre BROOKFIELD, mobile 4, à une vitesse de rotation de 100 tours/minute, dans une solution d'eau à 2 % et à 25 °C allant de 5000 à 40000 mPa.s (5000 à 40000 cPs) et plus particulièrement de 6500 à 35000 mPa.s (6500 à 35000 cPs).

**9.** Composition selon l'une quelconque des revendications 1 à 8, caractérisée par le fait que le poly(acide 2-acrylamido 2-méthylpropane sulfonique) réticulé est présent dans dans des concentrations allant préférentiellement de 0,01 à 20% en poids par rapport au poids total de la composition et plus préférentiellement de 0,1 à 10% en poids.

**10.** Composition selon l'une quelconque des revendications 1 à 9, caractérisée par le fait que les précurseurs d'actif sont choisis dans le groupe constitué par les phosphates d'actif ; les sulfates d'actif ; les esters alkylés ou acylés d'actif ; les éthers acylés ou alkylés d'actif, les amides d'actif et les dérivés d'ose d'actif ainsi que leurs mélanges.

**11.** Composition selon l'une quelconque des revendications 1 à 10, caractérisée par le fait que les précurseurs d'actif sont choisis dans le groupe constitué par les précurseurs de vitamine ; les précurseurs d'hydroxyacide ; les précurseurs de quercétine ; les précurseurs de nucléotide ; les hydroxyacétones phosphatées ; les précurseurs de glycérol ainsi que leurs mélanges.

**12.** Composition selon l'une quelconque des revendications 1 à 11, caractérisée par le fait que les précurseurs d'actif sont choisis dans le groupe constitué par les phosphates, les sulfates, les esters d'acide organique et les dérivés d'ose de vitamine A, C ou E ainsi que leurs mélanges.

**13.** Composition selon l'une quelconque des revendications 1 à 12, caractérisée par le fait que les précurseurs d'actif sont choisis dans le groupe constitué par les dérivés phosphatés de la vitamine C.

**14.** Composition selon l'une quelconque des revendications 1 à 13, caractérisée par le fait que les précurseurs d'actif sont choisis parmi les sels d'ascorbylphosphate de métal de transition.

**15.** Composition selon l'une quelconque des revendications 1 à 14, caractérisée par le fait que les précurseurs d'actif sont présents dans une concentration allant de 0,01% à 10 % en poids, et de préférence de 0,01 % à 1 % en poids par rapport au poids total de la composition.

**16.** Composition selon l'une quelconque des revendications 1 à 15, caractérisée en ce que le milieu cosmétiquement et/ou dermatologiquement acceptable est constitué d'eau ou d'eau et d'au moins un solvant organique choisi dans le groupe constitué par les solvants organiques hydrophiles, les solvants organiques lipophiles, les solvants amphiphiles ou leurs mélanges.

**17.** Composition selon la revendication 16, caractérisée en ce que les solvants organiques sont choisis dans le groupe constitué par les alcools mono- ou polyfonctionnels, les polyéthylène glycols éventuellement oxyéthylénés, les esters de propylène glycol, le sorbitol et ses dérivés, les di- alkyles d'isosorbide, les éthers de glycol et les éthers de propylène glycol, les esters gras.

**18.** Composition selon la revendication 16 ou 17, caractérisée en ce que le ou les solvants organiques représentent de 5 % à 98 % du poids total de la composition.

**19.** Composition selon l'une quelconque des revendications 1 à 18, caractérisée en ce qu'elle comprend en plus au moins une phase grasse.

**20.** Composition selon la revendication 19, caractérisée en ce que la phase grasse représente de 0 à 50% du poids total de la composition.

**21.** Composition selon l'une quelconque des revendications 1 à 20, caractérisée par le fait qu'elle contient en plus au moins un additif choisi dans le groupe constitué par les gélifiants et/ou épaississants classiques hydrophiles ou lipophiles ; des actifs hydrophiles ou lipophiles ; des conservateurs ;des antioxydants ; des parfums ; des émulsionnants ; des agents hydratants ; des agents pigmentants ; des dépigmentants ; des agents kératolytiques ; des vitamines ; des émollients ; des sequestrants ; des tensio-actifs ; des polymères ; des agents alcanisants ou acidifiants ; des charges ; des agents anti-radicaux libres ; des céramides ; des filtres solaires (notamment ultraviolets), des répulsifs pour insectes; des agents amincissants. ; des matières colorantes ; des bactéricides ; des antipelliculaires.

**22.** Utilisation non-thérapeutique de la composition selon l'une quelconque des revendications 1 à 21, comme produit capillaire rincé ou non-rincé pour le lavage, le soin, le conditionnement, le maintien de la coiffure ou la mise en forme des cheveux.

**23.** Utilisation non-thérapeutique de la composition selon l'une quelconque des revendications 1 à 21, comme produit de soin et/ou d'hygiène.

**24.** Utilisation non-thérapeutique de la composition selon l'une quelconque des revendications 1 à 21, comme produit de maquillage.

**25.** Utilisation non-thérapeutique de la composition selon l'une quelconque des revendications 1 à 21, comme produit antisolaire.

**26.** Utilisation non-thérapeutique de la composition selon l'une quelconque des revendications 1 à 21, comme produit de soin bucco-dentaire.

**27.** Procédé de traitement non-thérapeutique cosmétique de la peau, du cuir chevelu, des cheveux, des cils, des sourcils, des ongles ou des muqueuses, caractérisé par le fait qu'on applique sur le support une composition telle que définie selon l'une quelconque des revendications 1 à 21.

**28.** Utilisation de la composition selon l'une quelconque des revendications 1 à 21 pour préparer une formulation destinée à traiter thérapeutiquement la peau, le cuir chevelu ou les muqueuses.

## Claims

**1.** Cosmetic and/or dermatological composition, characterized in that it contains, in a cosmetically acceptable medium, at least one active principle precursor capable of releasing an active principle by enzymatic reaction on contact with the *stratum corneum* and at least one crosslinked poly(2-acrylamido-2-methylpropanesulphonic acid) neutralized to at least 90%, comprising, distributed randomly:

a) from 90 to 99.9% by weight of units of following general formula (1):

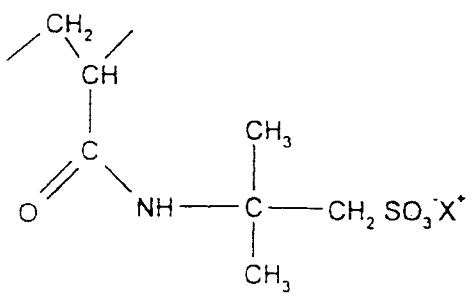

(1)

in which $X^+$ denotes a cation or a mixture of cations, it being possible for at most 10 mol% of the cations $X^+$ to be protons $H^+$;

b) from 0.01 to 10% by weight of crosslinking units resulting from at least one monomer having at least two olefinic double bonds, the proportions by weight being defined with respect to the total weight of the polymer.

**2.** Composition according to Claim 1, characterized in that the crosslinked poly(2-acrylamido-2-methylpropanesulphonic acid) contains a number of units of formula (1) in an amount which is sufficiently high to produce a hydrodynamic volume of the polymer in solution in water having a radius ranging from 10 to 500 nm, with a homogeneous and unimodal distribution.

**3.** Composition according to either one of Claims 1 and 2, characterized in that the crosslinked poly(2-acrylamido-2-methylpropanesulphonic acid) contains from 98 to 99.5% by weight of units of formula (1) and from 0.2 to 2% by weight of crosslinking units.

**4.** Composition according to any one of Claims 1 to 3, characterized in that, in the formula (1), the cation $X^+$ is $NH_4^+$.

**5.** Composition according to any one of Claims 1 to 4, characterized in that the crosslinking monomers correspond to the following general formula (2):

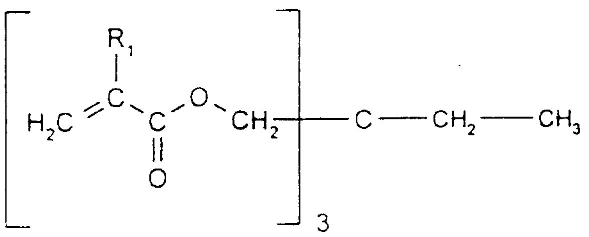

(2)

in which $R_1$ denotes a hydrogen atom or a $C_1$-$C_4$ alkyl.

6. Composition according to any one of Claims 1 to 5, characterized in that the poly(2-acrylamido-2-methylpropanesulphonic acid) is crosslinked with trimethylolpropane triacrylate.

7. Composition according to any one of Claims 1 to 6, characterized in that the crosslinked poly(2-acrylamido-2-methylpropanesulphonic acid) exhibits a viscosity, measured with a Brookfield viscometer, rotor 4, at a rotational speed of 100 revolutions/minute in a 2% solution in water at 25°C, of greater than or equal to 1000 mPa.s (1000 cPs).

8. Composition according to any one of Claims 1 to 7, characterized in that the crosslinked poly(2-acrylamido-2-methylpropanesulphonic acid) exhibits a viscosity, measured with a Brookfield viscometer, rotor 4, at a rotational speed of 100 revolutions/minute in a 2% solution in water at 25°C, ranging from 5000 to 40,000 mPa.s (5000 to 40,000 cPs) and more particularly from 6500 to 35,000 mPa.s (6500 to 35,000 cPs).

9. Composition according to any one of Claims 1 to 8, characterized in that the crosslinked poly(2-acrylmido-2-methylpropanesulphonic acid) is present in concentrations preferentially ranging from 0.01 to 20% by weight with respect to the total weight of the composition and more preferentially from 0.1 to 10% by weight.

10. Composition according to any one of Claims 1 to 9, characterized in that the active principle precursors are chosen from the group composed of active principle phosphates; active principle sulphates; active principle alkyl or acyl esters; active principle acyl or alkyl ethers; active principle amides and active principle monosaccharide derivatives, and their mixtures.

11. Composition according to any one of Claims 1 to 10, characterized in that the active principle precursors are chosen from the group composed of vitamin precursors; hydroxy acid precursors; quercetin precursors; nucleotide precursors; phosphated hydroxyacetones; glycerol precursors, and their mixtures.

12. Composition according to any one of Claims 1 to 11, characterized in that the active principle precursors are chosen from the group composed of phosphates, sulphates, organic acid esters and the monosaccharide derivatives of vitamin A, C or E, and their mixtures.

13. Composition according to any one of Claims 1 to 12, characterized in that the active principle precursors are chosen from the group composed of the phosphate derivatives of vitamin C.

14. Composition according to any one of Claims 1 to 13, characterized in that the active principle precursors are chosen from the transition metal salts of ascorbylphosphate.

15. Composition according to any one of Claims 1 to 14, characterized in that the active principle precursors are present in a concentration ranging from 0. 01% to 10% by weight and preferably from 0. 01% to 1% by weight with respect to the total weight of the composition.

16. Composition according to any one of Claims 1 to 15, characterized in that the cosmetically and/or dermatologically acceptable medium is composed of water or of water and of at least one organic solvent chosen from the group composed of hydrophilic organic solvents, lipophilic organic solvents, amphiphilic solvents or their mixtures.

17. Composition according to Claim 16, characterized in that the organic solvents are chosen from the group composed of mono- or polyfunctional alcohols, optionally oxyethylenated polyethylene glycols, propylene glycol esters,

sorbitol and its derivatives, dialkyl isosorbides, glycol ethers and propylene glycol ethers, or fatty esters.

**18.** Composition according to Claim 16 or 17, characterized in that the organic solvent or solvents represent from 5% to 98% of the total weight of the composition.

**19.** Composition according to any one of Claims 1 to 18, characterized in that it additionally comprises at least one fatty phase.

**20.** Composition according to Claim 19, characterized in that the fatty phase represents from 0 to 50% of the total weight of the composition.

**21.** Composition according to any one of Claims 1 to 20, characterized in that it additionally contains at least one additive chosen from the group composed of conventional hydrophilic or lipophilic gelling and/or thickening agents; hydrophilic or lipophilic active principles; preservatives; antioxidants; fragrances; emulsifiers; moisturizing agents; pigmenting agents; depigmenting agents; keratolytic agents; vitamins; emollients; sequestering agents; surfactants; polymers; basifying or acidifying agents; fillers; agents for combatting free radicals; ceramides; sunscreen agents (in particular ultraviolet screening agents); insect repellents; slimming agents; colouring materials; bactericides; or antidandruff agents.

**22.** Non-therapeutic use of the composition according to any one of Claims 1 to 21, as rinse-out or leave-in hair product for washing, caring for, conditioning or form retention of the hairstyle or shaping the hair.

**23.** Non-therapeutic use of the composition according to any one of Claims 1 to 21, as care and/or hygiene product.

**24.** Non-therapeutic use of the composition according to any one of Claims 1 to 21, as make-up product.

**25.** Non-therapeutic use of the composition according to any one of Claims 1 to 21, as anti-sun product.

**26.** Non-therapeutic use of the composition according to any one of Claims 1 to 21, as oral care product.

**27.** Process for the non-therapeutic cosmetic treatment of the skin, scalp, hair, eyelashes, eyebrows, nails or mucous membranes, characterized in that a composition as defined according to any one of Claims 1 to 21 is applied on the substrate.

**28.** Use of the composition according to any one of Claims 1 to 21 for preparing a formulation intended for the therapeutic treatment of the skin, scalp or mucous membranes.

**Patentansprüche**

**1.** Kosmetische und/oder dermatologische Zusammensetzung, dadurch gekennzeichnet, daß
sie in einem kosmetisch akzeptablen Medium mindestens einen Wirkstoffvorläufer, der durch eine im Kontakt mit dem *Stratum corneum* ablaufende enzymatische Reaktion einen Wirkstoff freizusetzen vermag, und mindestens eine vernetzte und zu mindestens 90 % neutralisierte Poly(2-acrylamido-2-methylpropansulfonsäure) enthält, die zufällig verteilt enthält:

a) 90 bis 99,9 Gew.-% Einheiten der folgenden allgemeinen Formel (1)

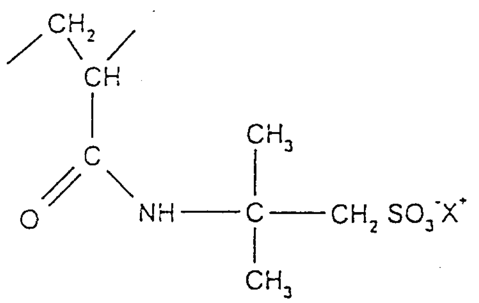

(1)

in der $X^+$ ein Kation oder ein Kationengemisch bedeutet, wobei höchstens 10 Mol-% der Kationen $X^+$ Protonen $H^+$ sein können,

b) 0,01 bis 10 Gew.-% vernetzende Einheiten, die von mindestens einem Monomer stammen, das mindestens zwei olefinische Doppelbindungen aufweist, wobei die Gewichtsanteile bezogen auf das Gesamtgewicht des Polymers angegeben sind.

2. Zusammensetzung nach Anspruch 1, dadurch gekennzeichnet, daß die vernetzte Poly(2-acrylamido-2-methylpropansulfonsäure) Einheiten der Formel (1) in einer Menge enthält, die ausreicht, um ein hydrodynamisches Volumen des Polymers in wäßriger Lösung zu erhalten, das einen Radius von 10 bis 500 nm aufweist und dessen Verteilung homogen und unimodal ist.

3. Zusammensetzung nach einem der Ansprüche 1 und 2, dadurch gekennzeichnet, daß die vernetzte und zu mindestens 90 % neutralisierte Poly(2-acrylamido-2-methylpropansulfonsäure) 98 bis 99,5 Gew.-% Einheiten der Formel (1) und 0,2 bis 2 Gew.-% vernetzende Einheiten enthält.

4. Zusammensetzung nach einem der Ansprüche 1 bis 3, dadurch gekennzeichnet, daß das Kation $X^+$ in der Formel (1) $NH_4^+$ ist.

5. Zusammensetzung nach einem der Ansprüche 1 bis 4, dadurch gekennzeichnet, daß die vernetzenden Monomere die folgende allgemeine Formel (2)

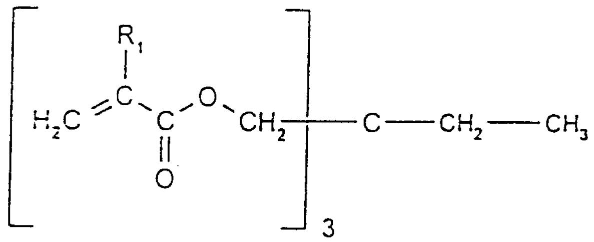

(2)

aufweisen, in der $R_1$ Wasserstoff oder ein $C_{1-4}$-Alkyl bedeutet.

6. Zusammensetzung nach einem der Ansprüche 1 bis 5, dadurch gekennzeichnet, daß die Poly(2-acrylamido-2-methylpropansulfonsäure) mit Trimethylolpropantriacrylat vernetzt ist.

7. Zusammensetzung nach einem der Ansprüche 1 bis 6, dadurch gekennzeichnet, daß die vernetzte Poly(2-acrylamido-2-methylpropansulfonsäure) eine mit einem BROOKFIELD-Viskosimeter, Drehkörper 4, bei einer Drehgeschwindigkeit von 100 U/min bei 25 °C in 2%iger wäßriger Lösung gemessene Viskosität größer als oder gleich 1000 mPas (1000 cP) aufweist.

8. Zusammensetzung nach einem der Ansprüche 1 bis 7, dadurch gekennzeichnet, daß die vernetzte Poly(2-acrylamido-2-methylpropansulfonsäure) eine mit einem BROOKFIELD-Viskosimeter, Drehkörper 4, bei einer Drehgeschwindigkeit von 100 U/min bei 25 °C in 2%iger wäßriger Lösung gemessene Viskosität von 5000 bis 40000 mPas

(5000 bis 40000 cP) und insbesondere 6500 bis 35000 mPas (6500 bis 35000 cP) aufweist.

9. Zusammensetzung nach einem der Ansprüche 1 bis 8, dadurch gekennzeichnet, daß die vernetzte Poly(2-acrylamido-2-methylpropansulfonsäure) in einer Konzentration von vorzugsweise 0,01 bis 20 Gew.-%, bezogen auf das Gesamtgewicht der Zusammensetzung, und noch bevorzugter 0,1 bis 10 Gew.-% enthalten ist.

10. Zusammensetzung nach einem der Ansprüche 1 bis 9, dadurch gekennzeichnet, daß die Wirkstoffvorläufer unter den Phosphaten des Wirkstoffs, den Sulfaten des Wirkstoffs, den Alkyl- oder Acylestern des Wirkstoffs, den Acyl- oder Alkylethern des Wirkstoffs, den Amiden des Wirkstoffs und den Zuckerderivaten des Wirkstoffs sowie deren Gemischen ausgewählt sind.

11. Zusammensetzung nach einem der Ansprüche 1 bis 10, dadurch gekennzeichnet, daß die Wirkstoffvorläufer unter Vitamin-Vorläufern, Hydroxysäure-Vorläufern, Quercetin-Vorläufern, Nucleotid-Vorläufern, phosphathaltigen Hydroxyacetonen, Glycerin-Vorläufern sowie deren Gemischen ausgewählt sind.

12. Zusammensetzung nach einem der Ansprüche 1 bis 11, dadurch gekennzeichnet, daß die Wirkstoffvorläufer unter den Phosphaten, den Sulfaten, den Estern organischer Säuren und den Zuckerderivaten von Vitamin A, C oder E sowie deren Gemischen ausgewählt sind.

13. Zusammensetzung nach einem der Ansprüche 1 bis 12, dadurch gekennzeichnet, daß die Wirkstoffvorläufer unter den phosphathaltigen Derivaten von Vitamin C ausgewählt sind.

14. Zusammensetzung nach einem der Ansprüche 1 bis 13, dadurch gekennzeichnet, daß die Wirkstoffvorläufer unter den Übergangsmetall-Ascorbylphosphatsalzen ausgewählt sind.

15. Zusammensetzung nach einem der Ansprüche 1 bis 14, dadurch gekennzeichnet, daß die Wirkstoffvorläufer in einer Konzentration von 0,01 bis 10 Gew.-% und vorzugsweise 0,01 bis 1 Gew.-%, bezogen auf das Gesamtgewicht der Zusammensetzung, enthalten sind.

16. Zusammensetzung nach einem der Ansprüche 1 bis 15, dadurch gekennzeichnet, daß das kosmetisch und/oder dermatologisch akzeptable Medium aus Wasser oder aus Wasser und mindestens einem organischen Lösungsmittel besteht, das unter hydrophilen organischen Lösungsmitteln, lipophilen organischen Lösungsmitteln, amphiphilen organischen Lösungsmitteln und deren Gemischen ausgewählt ist.

17. Zusammensetzung nach Anspruch 16, dadurch gekennzeichnet, daß die organischen Lösungsmittel unter einwertigen oder mehrwertigen Alkoholen, gegebenenfalls ethoxylierten Polyethylenglykolen, Propylenglykolestern, Sorbit und dessen Derivaten, Di-Alkylisosorbiden, Glykolethern und Propylenglykolethern, Fettestern ausgewählt sind.

18. Zusammensetzung nach Anspruch 16 oder 17, dadurch gekennzeichnet, daß das oder die organischen Lösungsmittel 5 bis 98 % des Gesamtgewichts der Zusammensetzung ausmachen.

19. Zusammensetzung nach einem der Ansprüche 1 bis 18, dadurch gekennzeichnet, daß sie zusätzlich mindestens eine Fettphase enthält.

20. Zusammensetzung nach Anspruch 19, dadurch gekennzeichnet, daß der Anteil der Fettphase 0 bis 50 % des Gesamtgewichts der Zusammensetzung beträgt.

21. Zusammensetzung nach einem der Ansprüche 1 bis 20, dadurch gekennzeichnet, daß sie zusätzlich mindestens einen Zusatz enthält, der ausgewählt ist unter herkömmlichen hydrophilen oder lipophilen Gelbildnern und/oder Verdickungsmitteln, hydrophilen oder lipophilen Wirkstoffen, Konservierungsmitteln, Antioxidantien, Perfums, Emulgatoren, Hydratisierungsmitteln, pigmentierenden Mitteln, depigmentierenden Mitteln, Keratolytika, Vitaminen, Weichmachern, Maskierungsmitteln, grenzflächenaktiven Stoffen, Polymeren, Alkalisierungsmitteln und Säuerungsmitteln, Füllstoffen, Mitteln gegen freie Radikale, Ceramiden, Lichtschutzfiltern (insbesondere UV-Filter), Repellentien gegen Insekten, schlanker machenden Mitteln, Färbemitteln, Bakteriziden, Antischuppenmitteln.

22. Nicht-therapeutische Verwendung der Zusammensetzung nach einem der Ansprüche 1 bis 21 als Haarbehandlungsmittel, das ausgespült wird oder nicht ausgespült wird, zum Waschen, für die Pflege, die Konditionierung, die Erhaltung der Frisur oder die Gestaltung der Haare.

23. Nicht-therapeutische Verwendung der Zusammensetzung nach einem der Ansprüche 1 bis 21 als Pflegeprodukt und/oder Hygieneprodukt.

24. Nicht-therapeutische Verwendung der Zusammensetzung nach einem der Ansprüche 1 bis 21 als Schminkprodukt.

25. Nicht-therapeutische Verwendung der Zusammensetzung nach einem der Ansprüche 1 bis 21 als Lichtschutzprodukt.

26. Nicht-therapeutische Verwendung der Zusammensetzung nach einem der Ansprüche 1 bis 21 als Produkt für die bucco-dentale Pflege.

27. Verfahren zur nicht-therapeutischen kosmetischen Behandlung der Haut, der Kopfhaut, der Haare, der Wimpern, der Augenbrauen, der Nägel oder der Schleimhäute, dadurch gekennzeichnet, daß auf den Träger eine wie in einem der Ansprüche 1 bis 21 definierte Zusammensetzung aufgetragen wird.

28. Verwendung der Zusammensetzung nach einem der Ansprüche 1 bis 21 für die Herstellung einer Formulierung, die für die therapeutische Behandlung der Haut, der Kopfhaut oder der Schleimhäute vorgesehen ist.